**Europäisches Patentamt**

**European Patent Office**

**Office européen des brevets**

(19)

(11) Publication number : **0 585 168 A2**

## (12) EUROPEAN PATENT APPLICATION

(21) Application number : **93402073.6**

(22) Date of filing : **18.08.93**

(51) Int. Cl.$^5$ : **A61K 35/32, A61K 37/02, A61L 27/00**

(30) Priority : **21.08.92 US 933290**

(43) Date of publication of application :
**02.03.94 Bulletin 94/09**

(84) Designated Contracting States :
**AT BE CH DE DK ES FR GB GR IE IT LI LU MC NL PT SE**

(71) Applicant : **Bristol-Myers Squibb Company**
**345 Park Avenue**
**New York, N.Y. 10154 (US)**

(72) Inventor : **Bruce, A. Gregory**
**2433 36th Avenue West**
**Seattle, Washington 98199 (US)**

Inventor : **Strong, D. Michael**
**18624 94th Avenue West**
**Edmonds, Washington 98020 (US)**
Inventor : **Kibblewhite, Douglas J.**
**4049 West 11th Avenue**
**Vancouver B.C. V6R 2L4 (CA)**
Inventor : **Gombotz, Wayne R.**
**6406 N.E. 129th Place**
**Kirkland Washington 98034 (US)**
Inventor : **Larrabee, Wayne F.**
**1656 East Garfield**
**Seattle, Washington 98112 (US)**
Inventor : **Purchio, Anthony F.**
**801 33rd Avenue N.E.**
**Seattle, Washington 98133 (US)**

(74) Representative : **Durand, Yves Armand Louis et al**
**Cabinet Z. Weinstein 20, Avenue de Friedland**
**F-75008 Paris (FR)**

(54) **Composition and methods for the generation of bone.**

(57)   Compositions and methods utilizing TGF-β and demineralized bone matrix are described that are capable of inducing the formation of useful, non-resorptive bone. Bone formation is stimulated by the synergistic activity of a TGF-β molecule, such as TGF-β1 or TGF-5β, with a HCl-treated demineralized bone matrix. Normal, hard non-resorptive bone development is stimulated and can be utilized in the treatment of fractures, skeletal defects, surgical repairs and skeletal reconstructions, such as in maxillofacial reconstructions.

EP 0 585 168 A2

TECHNICAL FIELD OF THE INVENTION

The present invention is directed to a composition and a method of generating useful, non-resorptive bone formation. The present invention can be utilized for the treatment of fractures, skeletal defects and maxillofacial reconstructions.

BACKGROUND OF THE INVENTION

Bone has a remarkable regenerative capacity; however, adverse conditions may affect the bony repair process promoting fibrogenesis at the expense of bone union. As a result, maxillofacial and orthopedic surgeons employ osteoinductive agents such as grafts and implants to restore continuity and contour defects which arise from trauma, infection and neoplasm. At present, autologous cancellous bone is the choice graft material employed for both craniomandibulofacial and long bone defects. Osteoinduction may be enhanced by combining autogenetic or allogenic bone with osteogenic progenitor cells from bone marrow (Lindholm T.S. et al. (1982) Clin. Orthop. 171:251; Simmons, D.J. et al. (1973) Clin. Orthop. 97:237) and applying these agents as composite graft material. However, despite their effectiveness, autologous bone grafts present a limited resource which is not suited for repair of multiple or massive defects and cannot be used ad infinitum in patients having multiple procedures. Furthermore, harvesting of autologous bone is often associated with pain, expense and morbidity in excess of the primary surgical procedure.

It is for these reasons that scientists have continued to explore alternative sources for bone restoration. Alloplastic materials have been used for decades to contour and reconstruct the facial skeleton; however success has been limited by infection, extrusion (Davies, P.K.B, et al. (1971) Br. J. Plast. Surg. 24:405), pressure erosion of bone (Jobe, R. et al. (1973) PRS 51:169), lack of implant stabilization (Brown, B.L. et al. (1979) Arch. Otolaryng. 105:605), chronic foreign body (giant cell) reaction (Brown, B.L. et al. (1979) Arch. Otolaryng. 105:605) and implant resorption (Brown, B.L. et al. (1979) Arch. Otolaryng. 105:605). These synthetic materials act as bone replacement; they do not regenerate new bone that is physiologically competent and physically equivalent to bone. Frustration with these materials has in part fueled interest in new models of osteoinduction-making consistent bone regeneration a fundamental requirement for any repair material.

The discovery that demineralized bone matrix (DBM) can induce new bone formation in heterotopic host sites (Urist, M.R. (1965) Science 150:893) has formed the foundation of current research in the science of osteoinduction. However, in many cases, bone resorption occurs with DBM implants. A recent study concluded that DBM had an unacceptably high resorption rate and should be used only where the implant is positioned in sites rich in primitive mesenchymal cells or bone forming cells (Toriumi et al. (1990) Arch. Otolaryng. Head Neck Surg. 116:676-680). Bone induction (Mulliken J.B. et al. (1984) J. Surg. Res. 37:487) is believed to be mediated by diffusible protein substrate(s) which are "stored" in association with the noncollagenous bone matrix (Hauschka P.V. et al. (1986) J. Biol. Chem. 261:12665; Urist, M.R. et al. (1972) J. Dent. Res. 50:1392; Sampath T.K. et al. (1987) PNAS USA 84:7109). Liberation of compartmentalized "bone growth factors", results in the differentiation and stimulation of nearby osteocompetent cells. Responding osteogenic cells exist in the margins of fresh bone defects and in association with the endosteum and periosteum. Numerous non-osseous tissues are also capable of responding to these osteoinductive growth factors including connective tissue such as muscle and muscle fascia (Urist M.R. et al. (1969) Clin. Orth. 64:194), thymus (Friedenstein A.J. (1973) Determined and Inducible Osteogenic Precursor Cells In Hard Tissue Growth, Repair and Remineralization. Sognas R. and Vaughan J. (eds). Ciba Foundation Symposium II. Associated Scientific Publishers, Amsterdam, p. 169) and the connective tissue sheaths of the visceral organs (Chalmers J. et al. (1975) J. Bone Joint Surg. 57B:36). These tissues possess sufficient primitive, undifferentiated mesenchymal cells (Urist M.R. et al. (1969) Clin. Orthop. 64:194) which respond to osteoinductive factors by a change in phenotype and have been termed "inducible osteogenic precursor cells" Friedenstein A.J. (1973) Determined and Inducible Osteogenic Precursor Cells In Hard Tissue Growth, Repair and Remineralization. Sognas R. and Vaughan J. (eds). Ciba Foundation Symposium II. Associated Scientific Publishers, Amsterdam, p. 169). Cytodifferentiation of uncommitted mesenchymal cells involves a sequential series of events: mesenchymal cell proliferation and activation, cartilage formation, woven bone formation and finally, generation of lamellar bone. At present, several potential bone growth substances have been sequenced and are available for study including osteogenic, bone morphogenetic protein and transforming growth factor-beta.

Demineralized bone has been used in a variety of craniomandibulofacial applications, however, the amount of actual new bone growth has varied considerably (Mulliken J.B. (1985) Induced Osteogenesis and Craniofacial Surgery In Caronnie E. (ed.): Craniofacial Surgery. Little Brown, Boston; Toriumi D.M. et al. (1990) Arch. Otolaryngol. Head Neck Surg. 116:676). High resorption rates occur when demineralized bone is used in augmentation procedures over the nasal dorsal, malar eminence, supraorbital ridge, nasal tip and along the mandible. Long term follow-up of dorsal nasal implants has shown an unacceptable high rate of resorption ranging from 50.7% (ave. 13 mos.) for small implants to 82.5% (ave. 24 mos.) for larger implants (Toriumi D.M. et al. (1990) Head Neck Surg. 116:676). High rates of resorption are a particular problem when demineralized bone is used in solid form such as split rib, suggesting poor contact with the surrounding bone or limited diffusion of aggregate matrix protein into the nearby tissues. Resorption is also severe when implants are not applied to fresh bony elements. Pressure from overlying tissue contraction, ischemia and infection also favor implant resorption at a rate which exceeds osteoinduction.

In an effort to enhance the osteoinductive effect of bone derivative implants, the present invention has placed emphasis on the isolation, characterization and purification of matrix-bound protein growth factors responsible for osteoinduction. Several structurally related bone growth factors have now been isolated and sequenced (Wang E.A. et al. (1988) PNAS USA 85:9484; Wang E.A. et al. (1990) PNAS USA 85:2220; Ozkaynak E. et al. (1990) EMBO 9:2085), combined with biodegradable carriers for delivery and tested in experimental animal models. However, little consistency exists among investigators regarding the appropriate animal model for study of osteoinduction; eliminating the possibility for meaningful comparison of available data. The bone repair rate among experimental animals varies inversely with order along phylogenetic scale (Prolo, D.J. et al. (1982) Clin. Orthop. 108:230) and with the maturity (age) of the animal (Prolo, D.J. et al. (1982) Clin. Orthop. 108:230; Robinson R.A. (1971) J. Bone Joint Surg. 53A:1017). In fact, the majority of available osteoinductive proteins have been shown to have excellent bone forming capabilities when used in low order, immature species, having unlimited capacity for spontaneous healing. The quality of bone repair also depends on the anatomic location (Harris W.H. et al. (1968) J. Bone Joint Surg. 50A:1118) and size (Hjorting-Hansen E. et al. (1971) Br. J. Oral Surg. 9:33; Simmons D.J. (1980) Fracture Healing in Urist M.R. (ed.) Fundamental and Clinical Bone Physiology. J.B. Lippincott, Philadelphia, p. 291) of the defect. Wound size must be of significant proportion to preclude the possibility of spontaneous healing and to allow unequivocal evaluation of the osteogenic potential of an implant, graft or osteoinductive protein. An experimental model designed to meet these stringent requirements is the critical size defect (CSD) : defined as the smallest wound in a particular species and anatomic location that will not heal spontaneously over the lifetime of the animal (Schmitz J.P. et al. (1986) Clin. Orthop. Rel. Res. 205:299). A critical size defect heals by fibrous obliteration (Frame J.W. (1980) J. Oral Surg. 38:176) and not bone formation and is currently the best prototype available to study the process of maxillofacial and mandibular nonunion (Schmitz J.P. et al. (1986) Clin. Orthop. Rel. Res. 205:299). The calvarial critical size defect is considered the most severe test of osteoinductive capacity because the cranium is biologically relatively inert; corresponding to a deficiency in marrow and an absent nutrient blood supply (Prolo D.J. et al. (1982) Clin. Orthop. 108:230). Even a small defect in the human calvarium fails to heal spontaneously (Prolo D.J. et al. (1984) Neurosurgery 14:183). The calvarial CSD is a useful model for maxillofacial and mandibular nonunion because of their similar embryological derivation from a membrane precursor (Frame J.W. (1980) J. Oral Surg. 38:176). In addition, the physiologic state of the calvarium resembles atrophic mandibular bone (Bays R.A. (1983) Current Concepts In Bone Grafting in Irby W.B., Shelton D.W. (eds) Current Advances In Oral and Maxillofacial Surgery, Mosby, St. Louis, Vol. 4, p. 109) which is prone to fibrous nonunion. Critical size defect models in calvarial bone have been established in four animal species: rat, rabbit, dog and monkey. Frame (Frame J.W. (1980) J. Oral Surg. 38:176) has established the validity of the 15mm diameter CSD in the rabbit, which is identical to the primate (Hollinger J. et al. (1989) J. Oral Mixollofac. Surg. 47:1182). To date, osteoinductive and osteoconductive agents have failed to achieve consistent and reliable healing in the rabbit model.

TGF-β is a 24,000 Dalton polypeptide which modulates the growth and differentiation of many cell types. The latent TGF β in bone comprises the largest pool in the body by a factor of ten. It is a highly conserved protein, which suggests an essential role in cell physiology.

The human and monkey mature forms of TGF-β are identical. Most human cell types studied express both TGF-β and receptors for it (Bonewald L.F. et al. (1990) Clin. Orthop. 250:261-76). It is secreted in a latent form, bound to a large carrier protein. Its activation is the subject of intense investigation. Extremes in pH, and enzymes such as cathepsin D and plasmin have been identified as activators (Pfeilschifter J. et al. (J. Bone Mineral Res. 5(1):49-58).

In vitro experiments have shown that small quantities of TGF-β stimulate osteoblast-like cells to markedly increase the production of type I collagen, osteonectin, osteopontin, and fibronectin (Strong D.D. et al. (1991) J. Bone Mineral Res. 6(1):15-23). TGF-β inhibits osteoclasts, and osteoclasts activate bone-latent TGF-β. It has been proposed that the low pH and high enzyme activity found under the ruffled border of osteoclasts forms the ideal microenvironment for activation. Since osteoclasts activity is the primary event in bone remodeling, this could provide the trigger for activation of bone-latent TGF-β with subsequent osteoclast stimulation and new bone matrix production (Bonewald L.F. et al. (1990) Clin. Orthop. 250:261-76).

The need to develop an effective and nonresorptive induction of new bone led to the present invention. In this invention DBM and a TGF-β are combined to generate new bone which is capable of meeting the criteria of the CSD model and which overcomes the problems of bone resorption previously resultant from the use of DBM. New, hard, non resorptive bone is produced in this invention.

## SUMMARY OF THE INVENTION

The present invention is directed to compositions and methods for facilitating the growth and formation of hard, non-resorptive bone. Compositions of the present invention containing demineralized bone matrix and TGF-β can be utilized to stimulate the development of bone in areas of the body where new bone formation is desired.

Prior studies have been unable to produce lasting bone formation which did not undergo resorption. In this invention, the combination of agents utilized have effectively generated bone formation at skeletal defects, such as surgical holes in the skull, which commonly underwent little, if any, bone regeneration.

Methods for the stimulation of bone growth and for the treatment of fractures, skeletal defects, periodontal disorders and maxillofacial reconstruction are contemplated by the present invention.

The compositions and methods of this invention can be utilized for therapeutic, cosmetic, and surgical treatments ranging from osteoporosis to mandibular reconstruction to treatment of non-union bone fractures.

Other uses and applications of the present invention are contemplated that will be readily apparent to those skilled in the art, and the present invention is not limited to the above-stated illustrative applications.

## BRIEF DESCRIPTION OF THE DRAWINGS

FIGURE 1 illustrates the percent bone area determined for implants with each dose of TGF-β. A grid was applied over a representative area of an implant section stained with Goldner's stain. The percent bone area was determined as the percent of squares which contained green stain which indicates calcified bone.

FIGURE 2 illustrates the percent matrix area determined for implants with each dose of TGF-β. A grid was applied over a representative area of an implant section stained with Goldner's stain. The percent matrix area was determined as the percent of squares which contained the unstained residual demineralized bone matrix.

FIGURE 3 illustrates the osteiod surface area ($mm^2$) determined in implant sections with each dose of TGF-β. The sections were stained with Goldner's stain and the new bone which is not yet calcified, indicated by yellow staining, was quantitated using computer planimetric analysis.

FIGURE 4 illustrates the cumulative percent of TGF-β1 released from DBM implants over time. Samples were incubated in PBS with 1% BSA at 37° C while shaking. Two types of samples were prepared, one with a citric acid buffer, pH 2.5 and one with PBS pH 7.4.

FIGURE 5 illustrates the ELISA activity of TGF-β1 (conc. 100 μg/ml) incubated at 37° C in PBS with 1% HSA over time.

FIGURE 6 illustrates the cumulative percent of TGF-β1 (a) and TGF-5β (b) released from DBM implants over time. Samples were loaded with 1250, 250 or 100 μg TGF-β/g DBM and released under the same conditions described in Figure 4.

FIGURE 7 illustrates the total amount of TGF-β1, or TGF-5β extracted from DBM implants loaded with different amounts of TGF-β. Extractions were carried out in 4 M guanidine HCl, 1 mM N-ethylmaleimide, 10 mM EDTA at pH 6.8 for 16 hours at 4° C. After extraction samples were dialyzed against 0.2 M acetic acid and assayed by ELISA.

## DESCRIPTION OF PREFERRED EMBODIMENTS

The present invention is directed to a method for inducing the production of hard, non-resorptive bone growth. Bone growth is stimulated in this invention by a composition containing a demineralized bone matrix and a transforming growth factor beta.

Previous studies with material classified as demineralized bone has resulted in some bone growth which has been attributed to various bone growth factors, such as bone morphogenic protein (BMP-2), which were present in the bone powder. The bone formed from this powder had demonstrated a high rate of resorption and thus did not constitute a lasting, hard bone formation.

This problem was overcome by the present inventors in the utilization of a demineralized bone matrix (DBM) prepared by acid treatment and lyophilization. The DBM has low levels of growth factor activity. The factors detected in DBM are listed in TABLE 1. The DBM is operatively attached to a TGF-β. In a preferred embodiment, DBM is combined with a sufficient amount of TGF-β to result in an essentially irreversible binding of TGF-β to the DBM. When DBM and TGF-β are so combined and administered, osteoinduction occurs and new, hard non-resorptive bone is produced.

## TABLE 1

| GROWTH FACTOR | | CONCENTRATION ug/g Wet Bone Powder |
|---|---|---|
| (IGF-II) | Insulin-Like Growth Factor-II | 1.26 |
| (TGF-ß) | Transforming Growth Factor - ß | 0.46 |
| (IGF-I) | Insulin-Like Growth Factor - I | 0.085 |
| (PDGF) | Platelet-Derived Growth Factor | 0.067 |
| (FGF) | Fibroblast Growth Factor | 0.024 |
| (EGF) | Epidermal Growth Factor | Undetectable |

The DBM of the present invention is a bone derivative prepared by demineralizing bone with HCl, followed by lyophilization. The final powder is composed of particles ranging in size from 74 μm to 640 μm.

TGF-β is a 24 kD homodimeric protein that stimulates the migration, proliferation and matrix synthesis of mesenchymal cells. Bone is the largest reservoir of TGF-β in the body (S.M. Seyedin et al. (1986) J. Biol. Chem. 261:5693-5695) , and its ability to promote bone formation suggest that it may have potential as a therapeutic agent in diseases of bone loss (Noda, M. and Camilliere, J. (1989) Endocrinology 124:2991-2207). The in vivo application of TGF-β adjacent to periosteum has been shown to increase bone thickness and chondrogenesis (Joyce et al. (1990) J. Cell Biol. 110:2195; Marcelli et al. (1990) J. Bone Miner. Res. 5:1087-1096; Mackie E.J. and Trecksel, U. (1990) Bone 11:295-300). TGF-β has also been shown to induce bone closure of large bony defects in the skull (Beck et al. (1991) J. Bone and Miner. Res.6:1257-1265).

In the present invention the composition of TGF-β and DBM can be utilized for the treatment of fractures, in dental implants and for the treatment of structural defects, such as in maxillofacial reconstructions. Particularly useful embodiments of the present invention comprise the application of a formed matrix containing DBM and TGF-β to a body area where bone growth of a specified shape or form is desired. For example, a composition of this invention can be shaped and fitted in a patient to produce bone growth of a mandibular region of the jaw in a patient needing reconstructive surgery as a result of head and neck trauma or disease. The present invention can also encompass compositions and implants for the treatment of dental disorders, such as tooth loss and bone defects, (referred to herein as "dental implants").

A particularly useful composition of the present invention is present in a non-fluid, plastic malleable matrix which can be form-fitted to a site where bone growth is desired. New bone formation can then occur according to the shape and form of the matrix. This method can produce new bone which would resemble and replace or repair bony structures for reconstructive surgery, such as jaws, skull plates, cheek bones, collar bones, nonunion fractures, fingers, toes, etc.

In order to more clearly describe the present invention and its embodiments, the following definitions are included.

As used herein, the term "TGF-β" refers to any member of the family of transforming growth factor beta which include TGF-β1, TGF-β2, TGF-β3, TGF-β4, TGF-β5 as well as the TGF-β1/β2 hybrid molecules, designated TGF-5β.

A novel form of TGF-β, designated TGF-5β, comprises a hybrid amino acid residue sequence of TGF-β1 and TGF-β2. TGF-5β has more effective recombinant processing and expression than has been seen for either TGF-β1 or TGF-β2. Specific description of TGF-5β is disclosed in Madisen et al (1990) in Transforming Growth Factor β's. Chemistry. Biology and Therapeutics, Anal. N.Y. Acad. Sci. 593:7-25 and in U.S. patent application serial numbers 669,171, filed March 14, 1991, and 667,246, filed March 8, 1992, the disclosures of which are incorporated herein by reference.

The terms "unit dose" and "sufficient amount" and grammatical variations thereof, are used interchangeably and refer to physically discrete amounts of material, such as TGF-β and DBM, suitable as unitary dosages for patients, each unit contains a predetermined quantity of active material calculated to produce the desired therapeutic effect in association with the required diluent; that is, a carrier or vehicle. The specifications for the novel unit dose of this invention are dictated by, and are directly dependent upon, (a) the unique characteristics of the active material and the particular therapeutic effect to be achieved, and (b) the limitations inherent in the art of compounding such active material for therapeutic use.

As used herein the term "effective amount" means an amount sufficient to stimulate bone production and formation at the desired site in a patient. The effective amount for a particular patient may vary depending on such factors as the extent of the fracture, injury or defect, the overall health of the patient, the method of administration, the severity of any side effects, and the like.

The term "correspond" in its various grammatical forms, as used herein and in the claims in relation to the peptide sequences for TGF-β and any other peptides means the peptide sequence described plus or minus up to 3 amino acid residues at either or both of the amino and carboxy termini and containing only conservative substitutions in particular amino acid residues along the peptide and/or polypeptide sequence.

The term "conservative substitution" as used above denotes that one amino acid residue has been replaced by another, biologically similar residue. Examples of conservative substitutions include the substitutions of one hydrophobic residue such as Ile, Val, Leu, or Met for another, or the substitution of one polar residue for another such as between Arg and Lys, between Glu and Asp or between Gln and Asn, and the like.

In some instances the replacement of an ionic residue by an oppositely charged ionic residue such as Asp by Lys has been determined conservative in the art in that those ionic groups are thought to merely provide solubility assistance. In general, however, since the replacements discussed herein are on a relatively short synthetic peptide region, as compared to a whole protein, replacement of an ionic residue by another ionic residue of opposite charge is considered herein to be a "radical replacement" as are replacements by nonionic and ionic residues, and bulky residues such as Phe, Tyr or Trp and less bulky residues such as Gly, Ile and Val.

The terms "nonionic" and "ionic" residues are used herein in their usual sense to designate those amino acid residues that either bear no charge or normally bear a charge, respectively, at physiological pH value. Exemplary nonionic residues include Thr and Gln, while exemplary ionic residues include Arg and Asp.

Ferric salts of the present invention encompass any physiologically tolerable organic salts and particularly includes ferric citrate.

The phrases "pharmaceutically acceptable salts" and "physiologically tolerable salts", as used interchangeably herein, refer to non-toxic alkali metal, alkaline earth metal and ammonium salts used in the pharmaceutical industry, including the sodium, potassium, lithium, calcium, magnesium and ammonium salts and the like that are prepared by methods well-known in the art. The phrase also includes non-toxic acid addition salts that are generally prepared by reacting the compounds in this invention with a suitable organic or inorganic acid. Representative salts include the hydrochloride, hyrdrobromide, sulfate, bisulfate, acetate, oxalate, valerate, oleate, laureate, vorate, benzoate, lactate, phosphate, tosylate, citrate, maleate, fumarate, succinate, tartrate and the like.

As used herein, the term "operatively attached" refers to the linkage of groups in a manner such that the desired biological activity of the group is not inhibited by the attachment.

As used herein the term "operatively linked" refers to a linkage that does not interfere with the ability of either of the linked groups to function as described. In one preferred embodiment, DBM is operatively linked to a biologically active TGF-β compound in a manner that permits the TGF-β to interact with osteoblasts to stimulate bone formation in association with DBM and which does not interfere with the biological activity of the TGF-β. This allows TGF-β to be released when the DBM is absorbed by osteoclasts.

The TGF-β and DBM of the present invention are preferably present in a pharmaceutical composition together with one or more pharmaceutically acceptable carriers.

As used herein, the phrases "physiologically tolerable" and "pharmaceutically acceptable" are used interchangeably and refer to molecular entities and compositions that do not produce an allergic or similar untoward reaction, such as gastric upset, dizziness and the like, when administered to a patient.

A carrier is a material useful for administering the active compound and must be "acceptable" in the sense of being compatible with the other ingredients of the composition and not deleterious to the recipient thereof.

As used herein, the term "pharmaceutically acceptable carrier" refers to a compound which is compatible with administration to a patient and does not produce toxic or untoward effects upon such administration. Illustrative examples of pharmaceutically acceptable carriers are phosphate buffered saline, Ringer's solution, oils, gels, biodegradable matrices, including natural or synthetic polymers, and microspheres, as well as liposomes. Other pharmaceutically acceptable carriers are well known in the field of pharmacy and are contemplated by the present invention.

As used herein, the term "labeling agent" refers to a single atom or molecule that when operatively linked to a product of the present invention enables detection of its presence in an assay method. Illustrative labeling agents are fluorescent dyes, radioisotopes, enzymes and antibodies that can be either independently detected or detected in conjunction with the addition of a substrate or other molecule that reacts therewith.

As used herein the term "substantially" refers to a high level of similarity. A substantially purified peptide of the present invention refers to a preparation having less than about ten percent extraneous peptides present. A substantially similar sequence in the present invention has less than about ten percent variation with the reference sequence.

The pharmaceutical compositions are prepared by any of the methods well known in the art of pharmacy all of which involve bringing into association the active compound and the carrier therefor.

For therapeutic use, the agents utilized in the present invention can be administered in the form of conventional pharmaceutical compositions. Such compositions can be formulated so as to be suitable for parenteral administration, or as implants. In these compositions, the agents are typically dissolved or dispersed in a physiologically tolerable carrier.

As an example, the compounds of the present invention can be utilized in non-fluid malleable, plastic compositions such as sterile suspensions or putty-like matrices capable of being formed to fit a fracture or defect, or as isotonic preparations containing suitable preservatives. Particularly well suited for the present purposes are biodegradable malleable matrices constituted by aqueous isotonic and sterile semi-rigid materials which enable osteoblast migration into and bone formation in the shaped matrix

It should be understood that in addition to the aforementioned carrier ingredients the pharmaceutical formulation described herein can include, as appropriate, one or more additional carrier ingredients such as diluents, buffers, binders, surface active agents, thickeners, lubricants, preservatives (including antioxidants) and the like, and substances included for the purpose of rendering the formulation isotonic with the blood of the intended recipient.

As used herein, the term "about" refers to a range of values both greater than and/or less than the listed value by 10% or less. For example, a temperature of about 20°C will include temperature values of from 18°C to 22°C.

As used herein, the terms "plastic" and "malleable" are used interchangeably and refer to material which can be formed or molded into specified shapes and which can be made to maintain the formed shape. A particularly preferred support material of this invention comprises a biodegradable matrix containing DBM and TGF-β together with a degradable homopolymer or copolymer.

Illustrative homopolymers or copolymers include poly(lactic acid), poly(lactic-coglycolic acid), poly(glycolic acid), poly(caprolactone), poly(hydroxybutyric acid), poly(anhydrides), poly(orthoesters), poly(carbonates) poly(amides), poly(acetals), poly(amino acids) and poly(cyanoacrylates).

Transforming growth factor-β1 (TGF-β1) is a multifunctional regulator of cell growth and differentiation. It is capable of causing diverse effects such as inhibition of the growth of monkey kidney cells, (Tucker, R.F., G.D. Shipley, H.L. Moses & R.W. Holley (1984) Science 226:705-707) inhibition of growth of several human cancer cell lines, (Roberts, A.B., M.A. Anzano, L.M. Wakefield, N.S. Roches, D.F. Stern & M.B. Sporn (1985) Proc. Natl. Acad. Sci. USA 82:119-123; Ranchalis, J.E., L.E. Gentry, Y. Agawa, S.M. Seyedin, J. McPherson, A. Purchio & D.R. Twardzik (1987) Biochem. Biophys. Res. Commun. 148:783-789) inhibition of mouse keratinocytes, (Coffey, R.J., N.J. Sipes, C.C. Bascum, R. Gravesdeal, C. Pennington, B.E. Weissman & H.L. Moses (1988) Cancer Res. 48:1596-1602; Reiss, M. & C.L. Dibble (1988 In Vitro Cell. Dev. Biol. 24:537-544) stimulation of growth of AKR-2B fibroblasts (Tucker, R.F., M.E. Olkenant, E.L. Branum & H.L. Moses (1988) Cancer Res. 43:1581-1586) and normal rat kidney fibroblasts, (Roberts, A.B., M.A. Anzano, L.C. Lamb, J.M. Smith & M.B. Sporn (1981) Proc. Natl. Acad. Sci. USA 78:5339-5343) stimulation of synthesis and secretion of fibronectin and collagen, (Ignotz, R.A. & J. Massague (1986) J. Biol. Chem. 261:4337-4345; Centrella, M., T.L. McCarthy & E. Canalis (1987) J. Biol. Chem. 262:2869-2874) induction of cartilage-specific macromolecule production in muscle mesenchymal cells, (Seyedin, S.M., A.Y. Thompson, H. Bentz, D.M. Rosen, J. McPherson, A. Contin, N.R. Siegel, G.R. Galluppi & K.A. Piez (1986) J. Biol. Chem. 261:5693-5695) and growth inhibition of T and B lymphocytes. (Kehrl, J.H., L.M. Wakefield, A.B. Roberts, S. Jakeoview, M. Alvarez-Mon, R. Derynck, M.B. Sporn & A.S. Fauci (1986) J. Exp. Med. 163:1037-1050; Kehrl, J.H., A.B. Roberts, L.M. Wakefield, S. Jakoview, M.B. Sporn & A.S. Fauci (1987) J. Immunol. 137:3855-3860; Kasid, A., G.I. Bell & E.P. Director (1988) J. Immunol. 141:690-698; Wahl, S.M., D.A. Hunt, H.L. Wong, S. Dougherty, N. McCartney-Francis, L.M. Wahl, L. Ellingsworth, J.A. Schmidt, G. Hall, A.B. Roberts & M.B. Sporn (1988) J. Immunol. 140:3026-3032)

Recent investigations have indicated that TGF-β1 is a member of a family of closely related growth-modulating proteins including TGF-β2, (Seyedin, S.M., P.R. Segarini, D.M. Rosen, A.Y. Thompson, H. Bentz & J. Graycar (1987) J. Biol. Chem. 262:1946-1949; Cheifetz, S., J.A. Weatherbee, M.L.-S. Tsang, J.K. Anderson, J.E. Mole, R. Lucas & J. Massague (1987) Cell 48:409-415; Ikeda, T., M.M. Lioubin & H. Marquardt (1987) Biochemistry 26:2406-2410) TGF-β3, (TenDijke, P., P. Hansen, K. Iwata, C. Pieler & J.G. Foulkes (1988) Proc. Natl. Acad. Sci. USA 85:4715-4719; Derynck, R., P. Lindquist, A. Lee, D. Wen, J. Tamm, J.L. Graycar, L Rhee, A.J. Mason, D.A. Miller, R.J. Coffey, H.L. Moses & E.Y. Chen (1988) EMBO J. 7:3737-3743; Jakowlew, S.B., P.J. Dillard, P. Kondaiah, M.B. Sporn & A.B. Roberts (1988) Mol. Endocrinology. 2:747-755) TGF-β4, (Jakowlew, S.B., P.J. Dillard, M.B. Sporn & A.B. Roberts (1988) Mol. Endocrinology. 2:1186-1195) Mullerian inhibitory substance, (Cate, R.L., R.J. Mattaliano, C. Hession, R. Tizard, N.M. Faber, A. Cheung, E.G. Ninfa, A.Z. Frey, D.J. Dash, E.P. Chow, R.A. Fisher, J.M. Bertonis, G. Torres, B.P. Wallner, K.L. Ramachandran, R.C. Ragin, T.F. Manganaro, D.T. Maclaughlin & P.K, Donahoe (1986) Cell 45:685-698) and the inhibins. (Mason, A. J., J.S.

Hayflick, N. Ling, F. Esch, N. Ueno, S.-Y. Ying, R. Guillemin, H. Niall & P.H. Seeburg (1985) Nature 318:659-663)

TGF-β1 is a 24-kDa protein consisting of two identical disulfide-bonded 12 kD subunits. (Assoian, R.K., A. Komoriya, C.A. Meyers, D.M. Miller & M.B. Sporn (1983) J. Biol. Chem. 258:7155-7160; Frolik, C.A., L.L. Dart, C.A. Meyers, D.M. Miller & M.B. Sporn (1983) Proc. Natl. Acad. Sci. USA 80:3676-3680; Frolik, C.A., L.M. Wakefield, D.M. Smith & M.B. Sporn (1984) J. Biol. Chem. 259:10995-11000) Analysis of cDNA clones coding for human, (Derynck, R., J.A. Jarrett, E.Y. Chem, D.H. Eaton, J.R. Bell, R.K. Assoian, A.B. Roberts, M.B. Sporn & D.V. Goeddel (1985) Nature 316:701-705) murine, (Derynck, R., J.A. Jarrett, E.Y. Chem, & D.V. Goeddel (1986) J. Biol. Chem. 261:4377-4379) and simian (Sharples, K., G.D. Plowman, T.M. Rose, D.R. Twardzik & A.F. Purchio (1987) DNA 6:239-244) TGF-β1 indicates that this protein is synthesized as a larger 390 amino acid pre-pro-TGF-β1 precursor; the carboxyl terminal 112 amino acid portion is then proteolytically cleaved to yield the TGF-β1 monomer.

The simian TGF-β1 cDNA clone has been expressed to high levels in Chinese hamster ovary (CHO) cells. Analysis of the proteins secreted by these cells using site-specific antipeptide antibodies, peptide mapping, and protein sequencing revealed that both mature and precursor forms of TGF-β were produced and were held together, in part, by a complex array of disulfide bonds. (Gentry, L.E., N.R. Webb, J. Lim, A.M. Brunner, J.E. Ranchalis, D.R. Twardzik, M.N. Lioubin, H. Marquardt & A.F. Purchio (1987) Mol. Cell Biol. 7:3418-3427; Gentry, L.E., M.N. Lioubin, A.F. Purchio & H. Marquardt (1988) Mol. Cell. Biol. 8:4162-4168) Upon purification away from the 24kD mature rTGF-β1, the 90 to 110 kD precursor complex was found to consist of three species: pro-TGFβ1, the pro-region of the TGF-β1 precursor, and mature TGF-β1. (Gentry, L.E., N.R. Webb, J. Lim, A.M. Brunner, J.E. Ranchalis, D.R. Twardzik, M.N. Lioubin, H. Marquardt & A.F. Purchio (1987) Mol. Cell Biol. 7:3418-3427; Gentry, L.E., M.N. Lioubin, A.F. Purchio & H. Marquardt (1988) Mol. Cell. Biol. 8:4162-4168) Detection of optimal biological activity required acidification before analysis, indicating that rTGF-β1 was secreted in a latent form.

The pro-region of the TGF-β1 precursor was found to be glycosylated at three sites (Asn 82, Asn 136, and Asn 176) and the first two of these (Asn 82 and Asn 136) contain mannose-6-phosphate residues. (Brunner, A.M., L.E. Gentry, J.A. Cooper & A.F. Purchio (1988) Mol. Cell Biol. 8:2229-2232; Purchio, A.F., J.A. Cooper, A.M. Brunner, M.N. Lioubin, L.E. Gentry, K.S. Kovacina, R.A. Roth & H. Marquardt (1988) J. Biol. Chem. 263:14211-14215) In addition, the rTGF-β1 precursor is capable of binding to the mannose-6-phosphate receptor and may imply a mechanism for delivery to lysosomes where proteolytic processing can occur. (Kornfeld, S. (1986) J. Clin. Invest. 77:1-6)

TGF-β2 is also a 24-kD homodimer of identical disulfide-bonded 112 amino acid subunits (Marquardt, H., M.N. Lioubin & T. Ikeda (1987) J. Biol. Chem. 262:12127-12131). Analysis of cDNA clones coding for human (Madisen, L., N.R. Webb, T.M. Rose, H. Marquardt, T. Ikeda, D. Twardzik, S. Seyedin & A.F. Purchio (1988) DNA 7:1-8; DeMartin, R., B. Plaendler, R. Hoefer-Warbinek, H. Gaugitsch, M. Wrann, H. Schlusener, J.M. Seifert, S. Bodmer, A. Fontana & E. Hoefer. EMBO J. 6:3673-3677) and simian (Hanks, S.K., R. Armour, J.H. Baldwin, F. Maldonado, J. Spiess & R.W. Holley (1988) Proc. Natl. Acad. Sci. USA 85:79-82) TGF-β2 showed that it, too, is synthesized as a larger precursor protein. The mature regions of TGF-β1 and TGF-β2 show 70% homology, whereas 30% homology occurs in the pro-region of the precursor. In the case of simian and human TGF-β2 precursor proteins differing by a 28 amino acid insertion in the pro-region; mRNA coding for these two proteins is thought to occur via differential splicing (Webb, N.R., L. Madisen, T.M. Rose & A.F. Purchio (1988) DNA 7:493-497).

The effects of TGF-β are thought to be mediated by the binding to specific receptors present on the surface of most cells (Massague, J. et al. (1985) J. Biol. Chem. 260:2636-2645; Segarini, P.R. et al. (1989) Mol. Endocrino. 3:261-272; Tucker, R.F., et al. (1984) Proc. Natl. Acad. Sci. USA 81:6757-6761; Wakefield, L.M., et al. (1987) J. Cell Biol. 105:965-975). Chemical crosslinking of [125I]-labeled TGF-β to cell surface components has identified three receptor size classes having molecule weights of 53-70 kDA (type I receptor), 80-120 kDa (type II receptor) and 250-350 kDa (type III receptor). The type I and II receptors have been implicated in signal transduction (Boyd, F.T. et al. (1989) J. Biol. Chem. 264:2272-2278; Laiho, M., et al. (1990) J. Biol. Chem. 265:18518-18524) while the type III receptor has been suggested to act as a storage protein (Segarini, P.R. et al. (1989) Mol. Endocrino. 3:261-272). Little is known concerning signal transduction mechanisms which occur after receptor-ligand interaction.

The pleiotrophic effects of TGF-β may be due to its ability to affect the transcription of other genes. TGF-β has been shown to induce *fos*, *myc* and *sis* in AKR-2B cells (Leof, E.B., et al. (1986) Proc. Natl. Acad. Sci. USA 83:1453-1458):1453-1458) enhance expression of c-*jun* B in A549 cells (Pertovaara, L., et al. (1989) Molecular and Cellular Biology 9:1255-1264), increase the mRNA for matrix proteins (Penttinen, R.P., et al. (1988) Proc. Natl. Acad. Sci. USA 85:1105-1110), IL-6 (Elias, J.A., et al. (1991) J. Immunol. 146:3437-3446) and EGF-receptors (Thompson, K.L. et al. (1988) J. Biol. Chem. 263:19519-19528) and decrease expression of PDGF receptor α subunits (Battegay, E. J., et al. (1990) Cell 63:515-524). It alters the pattern of integrin expression in osteosarcoma cells (Heino, J., et al. (1989) J. Biol. Chem. 264:21806-21813) and decreases the express of c-myc in keratinocytes (Coffey, R.J. et al. (1988b) Cancer Res. 48:1596-1602). TGF-β induces expression of IL-1β, TNF-α, PDGF and bFGF in human peripheral blood monocytes (McCartney-Francis, N., et al. (1991) DNA and Cell Biology 10:293-300).

The compositions and methods of the present invention can be utilized in therapeutic and reconstructive processes of bone formation such as regeneration and supplementation of osteoporotic bone, facial skeletal augmentation, reconstruction of mandibular and craniofacial defects, and periodontal applications.

The present invention contemplates a composition and method for the stimulation of growth of new bone and the regeneration of bone in fractures, repair of acetabular defects, osteoporosis, fillings for benign cystic lesions or tumor lesions of bone, and skeletal defects. Implants containing compositions of DBM and TGF-β, prepared under sterile conditions, can be implanted at bony sites where bone formation is desired. Sites at which such bone formation is contemplated include bone fracture, mandibular defects, cranial holes produced during surgery, tooth fillings and periodontal defects. The bone formed in the present inventions is vascularized and ossified, and is indistinguishable from naturally produced bone in structure and function. The bone formed in the present invention is not resorbed in contrast to prior bone formation stimulated by bone powder. The combination of DBM with TGF-β was found by the present inventors to produce hard, functional non-resorptive bone which is useful in the treatment of fractures, periodontal disorders and skeletal defects requiring the formation or regeneration of bone.

The methods and compositions of this invention are contemplated to be useful in plastic surgery, maxillofacial reconstruction, treatment of fractures, bone loss and skeletal defects and deformities. The osteogenic compositions containing DBM operatively attached to TGF-β is preferred to be in an embodiment which is plastic and malleable and capable of being formed into shapes and designs conducive to the above applications and uses. One contemplated use of these compositions is for the restructuring and reshaping of mandibular bone following maxillofacial reconstruction . Another contemplated use is for the treatment of non-union fractures. A third contemplated use is in dental implants for the production or regeneration of new bone and for the attachment of dental fixtures to the jaw. All uses consistent with the spirit and scope of the present invention are contemplated herein.

In one embodiment of this invention, it is contemplated that TGF-β is operatively linked to a carrier or matrix component, such as hydroxyapatite, in the composition with DBM. In this embodiment the TGF-β remains localized in the support matrix and bone formation is stimulated as a result of osteoblast interaction with DBM and the immobilized TGF-β.

Having generally described this invention, a further understanding can be obtained by reference to certain specific examples which are provided herein for purposes of illustration only and are not intended to be limiting unless otherwise specified.

## EXAMPLE 1

### PREPARATION OF DEMINERALIZED BONE MATRIX

Demineralized bone matrix (DBM) was prepared under sterile conditions from the harvested long bones of exsanguinated mature New Zealand White (NZW) rabbits. The bones were crushed, and cleaned by gentle agitation in warm sterile water and dried. The cleaned bones were then ground in a Tekmar bone mill to a particle size of 74 to 565 μm, assured by sieving. The ground bone was demineralized by repeated mixing with 0.6 M HCl, until no pH change occurred upon further washing with 0.6 M HCl. The pH was then adjusted to pH 7.27 with phosphate buffered saline, and the ground bone was lyophilized.

The processed DBM remained sterile. Repeated cultures of the DBM during processing were negative for bacteria and limulus endotoxin testing of the final product was negative.

## EXAMPLE 2

### PREPARATION OF IMPLANTS

Implants were prepared by reconstituting 300 mg DMB with 0.7 ml of 1 % rabbit serum albumin (Sigma Chemical Co., St. Louis, MO) in PBS to provide a thick paste. Either recombinant TGF-β1 or carrier solution was then added to the paste. Implants were prepared containing 0, 1, 10, 100 and 250 μg, respectively, of TGF-β1 per gram dry weight of DBM. The implants were shaped into cylinders approximately 7 mm in diameter and 25 mm long.

## EXAMPLE 3

CRANIAL OSTEOINDUCTION

Thirty-seven New Zealand White rabbits, 19 male and 18 female, weighing approximately 2.0 kg each were placed under quarantine and maintained for one week prior to the beginning of the study.

The rabbits were divided into four groups which received implants containing 1, 10, 100 and 250 μg/gram dry weight of DBM, respectively. Under sterile operating room conditions, anesthesia was administered to the rabbits by intramuscular injection of ketamine and xylazine. A 1 cm incision was made in the supraorbital area, and a subcutaneous pocket corresponding to the dimensions of the implant was dissected immediately above the periosteum, which was lightly abraded with a rasp. The implant was placed on the periosteum and the incision was closed. The 1 μg group received a second implant without TGF-β1 as a control.

The animals were fed a normal diet and housed in wire cages. Triple tetracycline labeling of bone formation was performed by administering intramuscular injections of tetracycline hydrochloride (20 mg/kg, Lederle Labs, Pearl River, NJ), demeclocycline (15 mg/kg, Lederle labs, Pearl River, NJ) and tetracycline hydrochloride (20 mg/kg) on days 16, 27 and 38, respectively. The animals were sacrificed at day 42 by intracardiac pentobarbital overdose. The implants were harvested intact in continuity with the underlying cranium. The isolated samples were tagged and placed in iced 10% buffered formaldehyde solution.

After dehydration in increasing concentrations of ethanol, the samples were embedded, undecalcified, in methyl methacrylate and sectioned in 8 mm thickness. Histomorphological study was then performed with Goldner's and acid phosphatase staining, and fluorescence microscopy for tetracycline labeling of the active osteoid mineralization front.

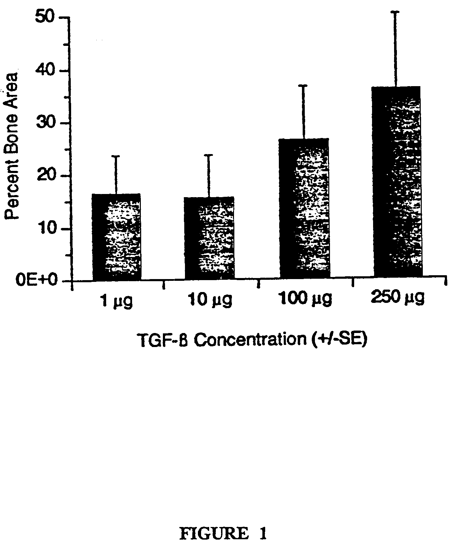

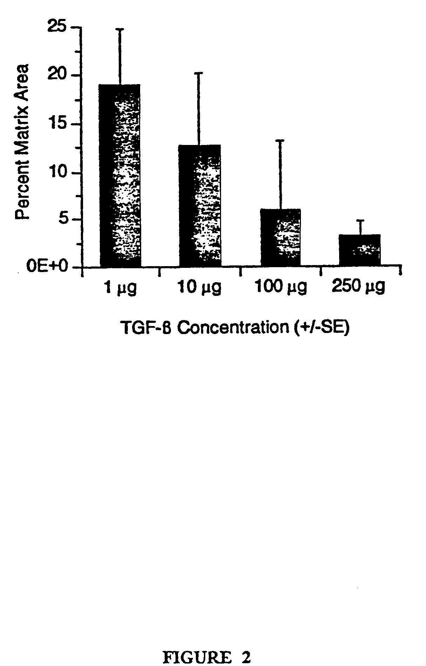

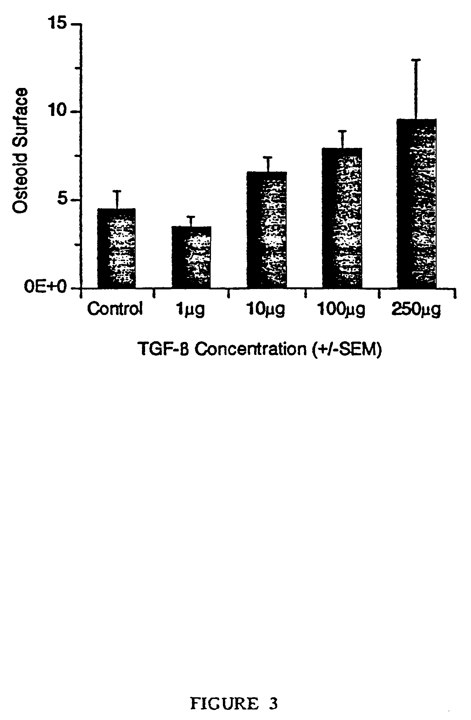

Gross examination revealed that both 100 μg and 250 μg implants were larger, darker and firmer than the controls. The histomorphometric data, illustrated in TABLE 2 and FIGURES 1, 2 and 3, showed significant increases in bone area per section and osteoid surface per bone surface. A significant reduction in DBM area per section was also seen. Upon cutting, the new bone had the consistency and appeance of trabecular bone, and was fused to the underlying calvarium. There were no changes observed in the overlying soft tissues. These results demonstrate that TGF-β1 at doses of 100 μg and 250 μg per gram of DBM significantly enhanced bone formation. DBM in the absence of TGF-β1 did not demonstrate significant osteoinductive activity.

## TABLE 2

| TGF-β DOSE | BONE AREA[#] | MATRIX AREA[#] | OSTEOID SURFACE[+] |
|---|---|---|---|
| 0 | 6.0 ± 2.4 | 7.2 ± 2.7 | 4.4 ± 3.2 |
| 1 | 6.0 ± 3.8 | 6.8 ± 4.5 | 3.5 ± 1.8 |
| 10 | 5.6 ± 3.3 | 3.7 ± 2.9 § | 6.6 ± 2.4 |
| 100 | 10.0 ± 4.8 § | 2.6 ± 1.8 § | 7.9 ± 3.2 § |
| 250 | 10.8 ± 2.4 § | 0.9 ± 0.8 § | 9.5 ± 6.8 § |

[#] Per Section          [+] Per bone surface;          § = p<0.05 vs. control

Similar studies were carried out using 250 μg implants of TGF-5β. The histomorphometric data is shown in Table 3 and appear simlar to the results obtained with TGF-β1.

## TABLE 3

| ARM | BONE AREA (mm$^2$) | MATRIX PARTICLE AREA (mm$^2$) | CORTEX AREA (mm$^2$) |
|---|---|---|---|
| Control | 14.9 ± 3.3 | 20.3 ± 1.4 | 14.7 ± 1.8 |
| 250 μg β1 | 55.3 ± 5.2 | 8.1 ± 1.3 | 13.8 ± 3.3 |
| 250 μg 5β | 70.9 ± 7.1 | 4.5 ± 2.0 | 15.7 ± 1.7 |

## EXAMPLE 4

### RELEASE OF TGF-β FROM IMPLANTS

Several in vitro release experiments have been carried out with the TGF-β/DBM implants used in the present invention.

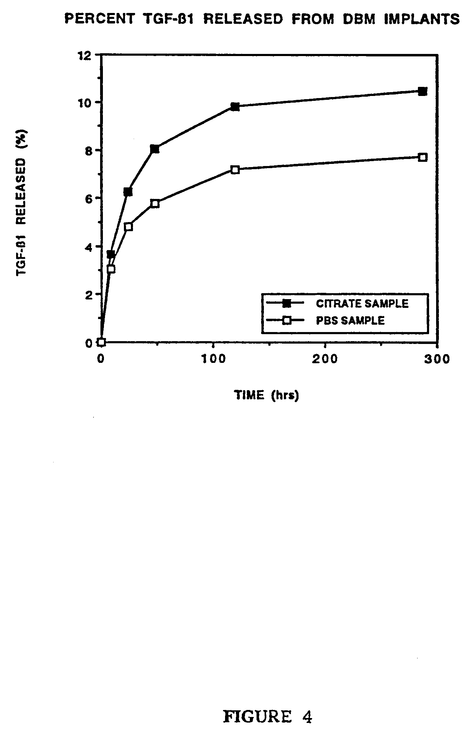

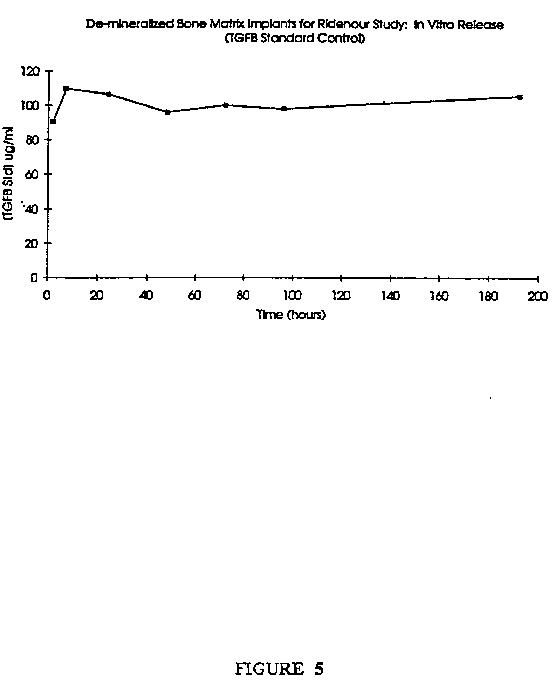

In the first study the TGF-β1/DBM implants containing 250 µg TGF-β/g DBM were incubated in PBS at 37° C while shaking. At specified times, the buffer was removed and replaced with a new buffer. The amount of TGF-β1 released from the implants into the buffer was determined by ELISA. Figure 4 shows the amount of TGF-β1 released over time from two different implants. One implant was prepared by mixing the DBM with TGF-β1 in a citrate buffer (pH 2.5) while other was made with a PBS buffer (pH 7.4). In both samples the most significant release of TGF-β1 occurred over the first 120 hours. The sample prepared with citrate buffer released approximately 10% of the TGF-β while the sample prepared in PBS released about 7%. In order to determine if the TGF-β was becoming inactivated in PBS at 37° C, a 100 µg/ml sample was incubated in the PBS and assayed by ELISA. Even after 180 hours no loss in ELISA binding was detected (FIGURE 5). Samples identical to those used in the release study were extracted in 4 M guanidine hydrochloride, 1 mM N-ethylmaleimide, 10 mM EDTA, pH 6.8 for 16 hours, to determine how much TGF-β1 was present in the implants. TABLE 4 shows that between 48 and 60% of the TGF-β could be recovered. The majority of the TGF-β1 is thus shown to remain bound to the DBM when exposed to physiological conditions. A DBM implant prepared with TGF-β1 in PBS was stored for 1 month at 4° C. After extraction, 55% of the TGF-β1 was recovered. This indicates that the TGF-β is stable in the DBM implants upon storage at 4° C.

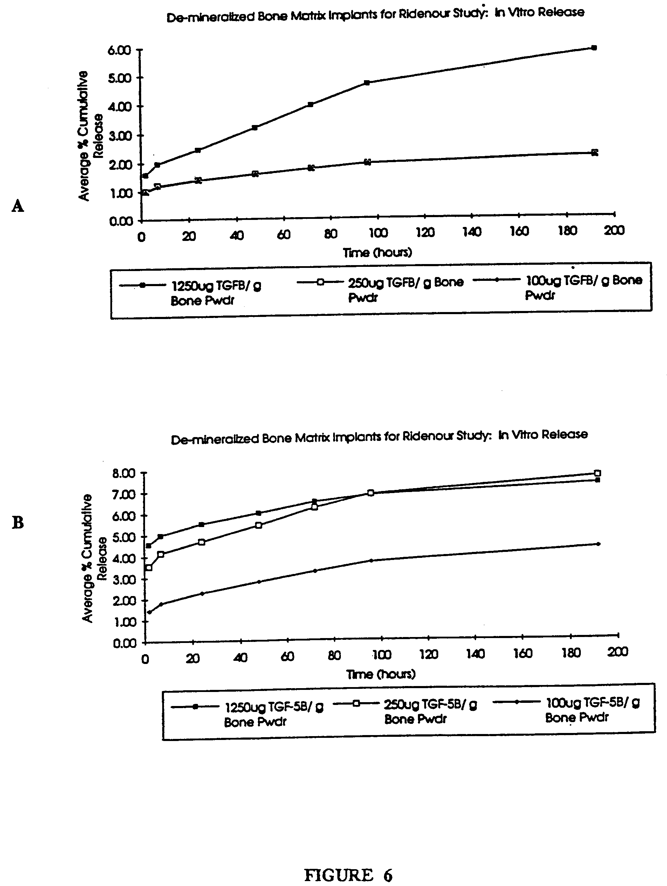

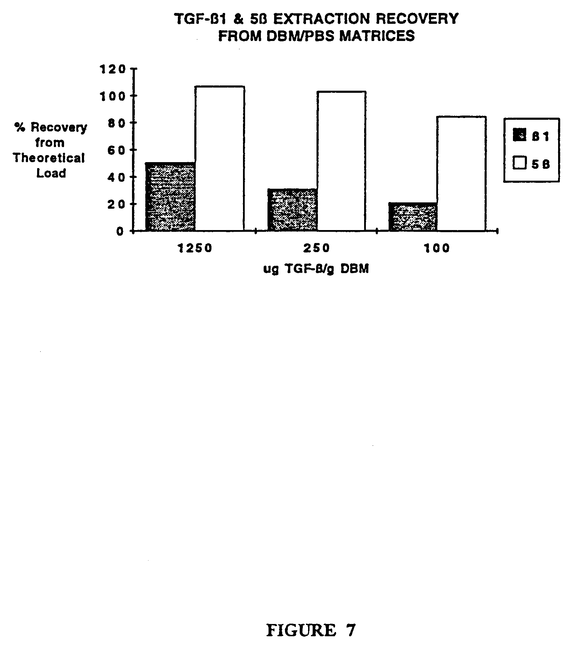

A second in vitro release study was done with DBM implants containing different concentrations of TGF-β1 and TGF-5β. The release kinetics are shown in FIGURES 6A and 6B. Again, only a small percentage of the TGF-β that is bound to the DBM is released. A greater percentage of the TGF-β1 is released from the 1250 µg TGF-β1/g DBM implants than from the samples containing lower loadings of TGF-β1. The implants containing TGF-5β exhibit a similar trend although both the 1250 and 250 µg TGF-5βg DBM samples release a greater percent of TGF-5β than the 100 µg TGF-5β/g DBM sample. These samples were also extracted in 4 M guanidine hydrochloride. FIGURE 7 shows that more TGF-5β was extracted from the DBM than TGF-β1. These results may indicated differences in DBM binding affinities between TGF-β1 and TGF-5β.

The results of the above studies show that only a small percentage of TGF-β1 or TGF-5β is released from DBM implants under physiological conditions, while more TGF-β is released from implants prepared with a citrate buffer (pH 2.5) than from implants prepared with PBS (pH 7.4). A greater percentage of TGF-5β can be released from or extracted from DBM than TGF-β1, suggesting that TGF-β1 is more tightly bound to DBM. The TGF-β1 remains stable in the DBM when stored at 4° C for up to one month.

**TABLE 4**

| SAMPLE | THEORETICAL RECOVERY (ng/ml) | ELISA RECOVERY (ng/ml) | PERCENTAGE |
|---|---|---|---|
| Stored 4°C 1 Month | 1548 | 850 | 55 |
| New Sample Made With PBS | 5161 | 2463 | 48 |
| New Sample Made With Citrate | 3132 | 1871 | 60 |

Samples extracted in 4 M Guanidine hydrochloride/1 mM N-ethylamaleimide/10 mM EDTA/pH 6.8 for 16 hours at 4°C. Dialyze against 0.2 M acetic acid for 24 hours.

The foregoing description and Examples are intended as illustrative of the present invention, but not as limiting. Numerous variations and modifications may be effected without departing from the true spirit and scope of the present invention.

## Claims

1. A composition useful in the stimulation of substantially non-resorptive bone growth comprising demineralized bone matrix operatively attached to an effective amount of transforming growth factor-beta and a pharmaceutically acceptable carrier.

2. The composition of Claim 1, wherein the transforming growth factor-beta is TGF-$\beta$1.

3. The composition of Claim 1, wherein the transforming growth factor-beta is TGF-5$\beta$.

4. The composition of Claim 1, wherein said composition is provided in a dental implant.

5. A method for stimulating bone growth comprising administering to a patient, at a site where bone growth is desired, a composition comprising demineralized bone matrix, transforming growth factor-beta and a pharmaceutically acceptable carrier such that osteoblasts can in association with said composition stimulate the production of bone.

6. The method of Claim 5, wherein said composition is a non-fluid, plastic composition capable of maintaining a specified form upon administration to said patient.

7. The method of Claim 5, wherein said composition is supplied to a patient at the site of a bone fracture, said composition occupying the space between and around said fracture.

8. The method of Claim 5, wherein said composition is applied to a surface of a medical implant or adjacent to said implant.

9. The method of Claim 5, wherein said composition is supplied as a dental implant.

10. The method of Claim 5, wherein said composition is supplied as a filling for cystic or tumor lesions of bone.

11. Use of a malleable plastic osteogenic composition, comprising demineralized bone matrix, transforming growth factor beta and a pharmaceutically acceptable carrier, in the treatment of bone fractures.

12. Use of a malleable plastic osteogenic composition, comprising demineralized bone matrix transforming growth factor beta which is TGF-5$\beta$ and a pharmaceutically acceptable carrier, in the treatment of bone fractures.

FIGURE 1

FIGURE 2

FIGURE 3

**PERCENT TGF-ß1 RELEASED FROM DBM IMPLANTS**

FIGURE 4

De-mineralized Bone Matrix Implants for Ridenour Study: In Vitro Release
(TGFB Standard Control)

FIGURE 5

De-mineralized Bone Matrix Implants for Ridenour Study: In Vitro Release

**A**

De-mineralized Bone Matrix Implants for Ridenour Study: In Vitro Release

**B**

FIGURE 6

TGF-ß1 & 5ß EXTRACTION RECOVERY
FROM DBM/PBS MATRICES

FIGURE 7